# EUROPÄISCHE PATENTANMELDUNG

(11) **EP 2 415 460 A1**
(43) Veröffentlichungstag der Anmeldung: **08.02.2012**
(21) Anmeldenummer: 10008104.1
(22) Anmeldetag: 03.08.2010
(51) Int. Cl.: A61K 9/00, A61K 31/197

(54) **Orale Darreichungsform von Pregabalin**

(71) Anmelder: ratiopharm GmbH, 89079 Ulm (DE)
(72) Erfinder: Meergans, Dominique, 81477 Munchen (DE); Paetz, Jana, 82272 Moorenweis (DE)
(74) Vertreter: Aechter, Bernd

(57) **Zusammenfassung**

Die Erfindung betrifft orale Darreichungsformen zur modifizierten Freisetzung von Pregabalin sowie Verfahren zu deren Herstellung.

## Beschreibung

Die Erfindung betrifft orale Darreichungsformen zur modifizierten Freisetzung von Pregabalin sowie Verfahren zu deren Herstellung.

Der IUPAC-Name von Pregabalin [INN] ist (S)-3-(Aminomethyl)-5-methylhexansäure. Die chemische Struktur von Pregabalin ist in nachstehender Formel (I) dargestellt:

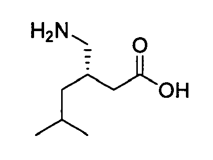

Pregabalin ist ein Analogon des physiologisch bedeutenden endogenen Neurotransmitters γ-Aminobuttersäure (GABA), der an der Regulation neuronaler Prozesse beteiligt ist. Es bindet an α2δ- Untereinheiten von Calciumkanälen. Die Synthese von Pregabalin und seine Verwendung als antikonvulsiv wirksame Substanz sind in EP 0 641 330 beschrieben.

Pregabalin ist in Europa derzeit zur Therapie von Epilepsie, neuropathischen Schmerzen und generalisierter Angststörung zugelassen. Ursachen für neuropathische Schmerzen können beispielsweise sein: diabetische Polyneuropathie, Post-Zoster-Neuralgie, Tumore, Chemotherapie, Trigeminusneuralgie, Alkoholmissbrauch, Vitamin-B-Mangel, Phantomschmerz, Borrelien-Infektion, komplexes regionales Schmerzsyndrom, Engpasssyndrom, Rückenschmerzen und AIDS. In den USA ist Pregabalin auch für die Behandlung von Fibromyalgie zugelassen.

Pregabalin wird unter dem Handelsnamen Lyrica^{®} in Form von sofort freisetzenden Tabletten vertrieben. Übliche therapeutische Dosierungen sind 150 mg bis 600 mg täglich, verteilt auf zwei oder drei Einzeldosen.

Zur Steigerung der Therapietreue des Patienten ("Compliance") und zur Erzielung möglichst konstanter Plasmakonzentrationen des Wirkstoffs ist eine Darreichungsform wünschenswert, die einmal täglich eingenommen werden kann.

Die Entwicklung einer derartigen geeigneten Formulierung wird jedoch dadurch erschwert, dass Pregabalin nicht im kompletten Gastrointestinaltrakt (GIT) resorbiert werden kann. Die Wirkstoffaufnahme erfolgt nur in den oberen Darmabschnitten. Das bedeutet, dass die Einführung einer Verzögerung der Freisetzung aus der Darreichungsform dazu führen würde, dass bei der Darmpassage große Wirkstoffmengen am Resorptionsfenster vorbeigeschleust würden. Daher ist eine Darreichungsform wünschenswert, die über eine längere Verweilzeit im oberen GIT verfügt und dabei den Wirkstoff kontinuierlich über einen ausreichend langen Zeitraum abgibt.

Die internationale Patentanmeldung WO 2007/052125 schlägt gastroretentive Formulierungen zur gesteuerten Freisetzung von Pregabalin vor. Die Pregabalinfreisetzung wird durch eine Matrixformulierung aus Polyvinylacetat und Polyvinylpyrrolidon gesteuert. Die Gastroretention wird dabei durch eine sofortige Vergrößerung der Darreichungsform im Magenmilieu erreicht, so dass die Formulierung aufgrund ihrer Größe den Pylorus (Magenpförtner) nicht mehr zu passieren in der Lage ist. Darüber hinaus wird offenbart, dass die Formulierung vorteilhaft zusammen mit der Nahrungsaufnahme und der daraus resultierenden längerer Verweilzeit im Magen anzuwenden ist. Ferner sei die Darreichungsform auch besonders geeignet nachts, da zu dieser Zeit die Magenaktivität üblicherweise verringert ist.

Die in dieser Formulierung eingesetzten Stoffe quellen schnell und stark auf. Dies birgt das Risiko vorzeitigen Quellens in der Speiseröhre, was zu deren Verstopfung führen kann. Ferner wäre eine von Nahrungsaufnahme und/oder Tageszeit unabhängige Formulierung wünschenswert.

Es war daher eine Aufgabe der vorliegenden Erfindung, eine Formulierung zur modifizierten Freisetzung von Pregabalin bereitzustellen, welche nicht beziehungsweise in erheblich geringerem Ausmaß das Risiko vorzeitigen Quellens in der Speiseröhre birgt. Es ist eine weitere beziehungsweise alternative Aufgabe der vorliegenden Erfindung, eine Formulierung bereitzustellen, die nicht auf eine verringerte Magenaktivität (z.B. nach Nahrungsaufnahme oder nachts) angewiesen ist.

Die Aufgaben werden durch das der erfindungsgemäßen oralen Darreichungsform zugrundeliegende gastroretentive Prinzip gelöst.

Ein Gegenstand der vorliegenden Erfindung ist eine orale Darreichungsform zur modifizierten Freisetzung von Pregabalin, umfassend
(a) Pregabalin
   in einer Matrix, die
(b) ein Quellmittel,
(c) einen Matrixbildner und
(d) ein Auftriebsmittel oder ein Sedimentationsmittel umfasst.

Der erfindungsgemäßen Darreichungsform liegt das Konzept zugrunde, eine gastroretentive Darreichungsform bereit zu stellen, die im Magen nicht nur quillt, sondern entweder im Magen obenauf schwimmt oder aber im Magen absinkt. Durch diese beiden Phänomene wird ein zu schnelles Passieren des Magentrakts verhindert, und zwar dabei auch unabhängig vom Ausmaß der Quellung.

Die Verwendung eines Auftriebmittels oder Sedimentationsmittels ermöglicht es mithin, ein weniger stark bzw. erheblich langsamer quellendes Quellmittel einzusetzen, so dass das Risiko eines Speiseröhrenverschlusses eliminiert beziehungsweise zumindest erheblich verringert werden kann.

Darüber hinaus wird durch den Auftrieb bzw. das Absinken eine Abhängigkeit von Nahrungsaufnahme beziehungsweise Schlaf zumindest verringert. Bevorzugt ist die orale Darreichungsform derart ausgebildet, dass ein Volumen der oralen Darreichungsform nach 10-minütigem Quellen in deionisiertem Wasser bei 37°C höchstens 25% größer ist als ein Volumen der Darreichungsform vor dem Quellen, bevorzugt höchstens 20%, höchstens 15% oder höchstens 12,5%. Das Volumen der Darreichungsform vor dem Quellen gibt das Volumen der Darreichungsform in trockenem Zustand bei Raumtemperatur und Normaldruck an und bildet hierin den Bezugspunkt für die Volumenzunahme.

Da orale Darreichungsformen der vorliegenden Art gewöhnlich mit Flüssigkeit eingenommen werden, wofür häufig Wasser gewählt wird, ist das Quellverhalten in Wasser in diesem Zusammenhang relevant. Bevorzugt liegt die Volumenzunahme aber auch bei Verwendung anderer Flüssigkeiten in dem angegebenen Bereich, etwa bei Einnahme mit Saft oder anderen Flüssigkeiten, die einen von Wasser verschiedenen pH-Wert aufweisen. Stellvertretend für diese Szenarien ist also bevorzugt die Volumenzunahme in Salzsäure im pH-Bereich von 1 bis 7 innerhalb des oben für Wasser angegebenen Zahlenbereichs, bevorzugt auch in Natronlauge im Bereich von pH 10 bis 7.

Unter deionisiertem Wasser wird vorliegend Wasser mit einer Leitfähigkeit bei 25 °C von 5 · 10⁻⁶ S/m oder weniger verstanden.

Die orale Darreichungsform enthält ein Quellmittel, das in Magensaft quellfähig ist.

Es ist in diesem Zusammenhang bevorzugt, dass ein Volumen der oralen Darreichungsform nach 45-minütigem Quellen in 0,1 N Salzsäure bei 37°C mindestens 7,5% größer ist als ein Volumen der Darreichungsform vor dem Quellen, bevorzugt mindestens 9 %, mindestens 10%, mindestens 12,5% oder mindestens 15%. Ferner ist bevorzugt, dass ein Volumen der oralen Darreichungsform nach 120-minütigem Quellen in 0,1 N Salzsäure bei 37°C mindestens 10% größer ist als ein Volumen der Darreichungsform vor dem Quellen, bevorzugt mindestens 12,5 %, mindestens 15%, mindestens 17,5% oder mindestens 20%.

Das Quellvolumen ist ein Indikator für das Quellverhalten im Magen. Je stärker die orale Darreichungsform zu quellen fähig ist, desto kleiner kann die orale Darreichungsform im trockenen Zustand sein, was für den Patienten hinsichtlich des Schluckverhaltens vorteilhaft ist.

Bevorzugt wird das maximale Quellvolumen gerade nicht innerhalb von 10, 20 oder auch 60 Minuten Quellen in oben genanntem HCl-Medium erreicht.

Das Auftriebsmittel dient dazu, der oralen Darreichungsform Auftrieb zu verschaffen, so dass diese an der Magensaftoberfläche bzw. im oberen Magenbereich treibt. Dies kann etwa dadurch erreicht werden, dass die orale Darreichungsform nach Kontakt mit der Magenflüssigkeit eine spezifische Dichte aufweist, die geringer ist als die der Magenflüssigkeit. Die geringe spezifische Dichte kann beispielsweise durch das Quellverhalten erreicht werden, oder aber durch die Entwicklung von Gas innerhalb der Darreichungsform, und insbesondere innerhalb der Matrix.

Getestet wird der Auftrieb mit Hilfe von 0,1 N Salzsäure. Die orale Darreichungsform steigt in 0,1 N Salzsäure eines Volumens von 1000 mL in einem Becherglas von 2000 mL Volumen bei 37°C vom Boden auf (bei unbewegter Flüssigkeit, d.h. ohne Rühren), besonders bevorzugt spätestens nach 30 Minuten, stärker bevorzugt nach spätestens 20 Minuten und noch stärker bevorzugt spätestens nach 10 oder 5 oder 3 Minuten.

Bevorzugt ist ein Auftriebsmittel, das geeignet ist, bei Kontakt mit 0,1 N Salzsäure Kohlendioxid und/oder Stickstoff freizusetzen. Idealerweise ist das Auftriebsmittel allgemein geeignet, bei Kontakt mit Salzsäure im pH-Bereich von 0 bis 6,5 Kohlendioxid und/oder Stickstoff freizusetzen.

Als Auftriebsmittel, welches auf der Funktion der Gasentwicklung basiert, kommen generell die im Fachbereich üblicherweise für Brausetabletten eingesetzten, Gas entwickelnden Sprengmittel in Betracht. Als Kohlenstoffdioxid freisetzende Auftriebsmittel kommen insbesondere pharmazeutisch verträgliche Carbonate und Hydrogencarbonate in Frage, insbesondere solche der Alkalimetalle, sowie Gemische davon. Beispielhaft sind Natriumhydrogencarbonat, Kaliumhydrogencarbonat, Kaliumcarbonat, Calciumhydrogencarbonat und Magnesiumhydrogencarbonat zu nennen. Natriumhydrogencarbonat ist dabei besonders bevorzugt.

Als Auftriebsmittel geeignet sind ferner Natriumglycincarbonat, eine Additionsverbindung von Natriumhydrogencarbonat und Glycin, sowie Arginincarbonat, die entsprechende Additionsverbindung von Arginin.

Mögliche Auftriebsmittel sind auch Ascorbinsäure, Weinsäure, Zitronensäure, deren pharmazeutisch verträgliche Salze und Kombinationen davon. Geeignet sind auch pharmazeutisch verträgliche Hydrogenphosphate wie zum Beispiel Natriumdihydrogenphosphat, Calciumhydrogenphosphat oder Kaliumdihydrogenphosphat.

Ferner können auch geeignete Kombinationen der genannten Auftriebsmittel eingesetzt werden.

Auftriebsmittel ist bzw. sind bevorzugt in einer Menge von 3 bis 15 Gew.% des Gesamtgewichts der oralen Darreichungsform enthalten, beispielsweise in Mengen von mindestens 3,5 Gew.-%, mindestens 4 Gew.-%, mindestens 5 Gew.-% oder mindestens 6 Gew.-% und/oder beispielsweise bis maximal 14 Gew.-%, maximal 13 Gew.-% oder maximal 10 Gew.-%. Eine zu hohe Menge Auftriebsmittel kann den Zusammenhalt der Matrix und damit die Funktion der oralen Darreichungsform gefährden.

Alternativ zum Auftriebsmittel kann ein Sedimentationsmittel eingesetzt werden. Ein solches Sedimentationsmittel dient der Beschwerung der aufgequollenen oralen Darreichungsform, so dass diese absinkt, im Idealfall bis auf den Magenboden.

Als Sedimentationsmittel kommen beispielsweise pharmazeutisch verträgliche anorganische Salze in Frage, beispielsweise Chloride, Sulfate, Phosphate und dergleichen. Beispiele hierfür wären Natriumchlorid, Calciumchlorid, Natriumsulfat, Calciumsulfat, Natriumphosphat oder Calciumphosphat. Besonders bevorzugt wird Natriumchlorid eingesetzt. Es können auch Kombinationen von Sedimentationsmitteln eingesetzt werden. Bevorzugt enthält die orale Darreichungsform Sedimentationsmittel in einer Menge von 3 bis 15 Gew.-% des Gesamtgewichts, beispielsweise in Mengen von mindestens 3,5 Gew.-%, mindestens 4 Gew.-%, mindestens 5 Gew.-% oder mindestens 6 Gew.-% und/oder beispielsweise bis maximal 14 Gew.-%, maximal 13 Gew.-% oder maximal 10 Gew.-%.

Ausführungsformen, die sowohl Auftriebsmittel als auch Sedimentationsmittel enthalten, sind zwar denkbar, erscheinen aber technisch wenig sinnvoll, es sei denn, beide wären in solchen Formen in der Matrix enthalten, dass sie zeitversetzt ihre Wirkung entfalten. Üblicherweise werden Sedimentationsmittel und Auftriebsmittel alternativ eingesetzt.

Der Matrixbildner dient vorwiegend der Bereitstellung eines Matrixgerüsts, das während der gewünschten Verweilzeit im Magen der oralen Darreichungsform und insbesondere der Matrix physikalische Stabilität verleiht beziehungsweise die Matrixbestandteile mechanisch zusammenhält.

Das Quellmittel dient vorwiegend dazu, durch Quellen in Kontakt mit dem Magensaft das Volumen der oralen Darreichungsform zu vergrößern.

In beispielhaften Ausführungsformen kann es sich bei Quellmittel und Matrixbildner um den gleichen Stoff beziehungsweise das gleiche Stoffgemisch handeln. Anders ausgedrückt kann ein Stoff beide Funktionen bereitstellen, es handelt sich dann um einen quellfähigen Matrixbildner.

In bevorzugten Ausführungsformen handelt es sich jedoch bei Quellmittel und Matrixbildner um unterschiedliche Stoffe beziehungsweise unterschiedliche Stoffgemische. Dann werden die beiden Funktionen durch unterschiedliche Stoffe bereitgestellt. Dies kann beispielsweise der Fall sein, wenn der Matrixbildner nicht oder nur wenig quellfähig ist. Dabei kann der Unterschied den Stoff an sich betreffen, etwa die chemische Zusammensetzung, wie sie sich etwa aus der formelmäßigen Darstellung ergibt. Beispielsweise im Falle von Polymeren können sich die Stoffe jedoch beispielsweise auch hinsichtlich Eigenschaften wie ihres Molekulargewichts, ihrer Dichte, ihrer Viskosität in Lösung oder dem Grad ihrer Quervernetzung unterscheiden. Bevorzugt handelt es sich um Stoffe, die deutlich verschieden sind, beispielsweise hinsichtlich ihrer Summenformel beziehungsweise Struktur oder ihrer physikalischen Eigenschaften.

Der Matrixbildner kann beispielsweise ein hydrophiler Matrixbildner sein. Als Matrixbildner kommen allgemein beispielsweise Polymere, Oligomere und Naturstoffe in Frage. Substanzen aus der Gruppe der Polysaccharide oder Alginate können als Matrixbildner zum Einsatz kommen. Aber auch Substanzen aus den Gruppen der Stärken oder auch Cellulose- Derivate wären hierfür geeignet. Der Matrixbildner kann einen oder mehrere Stoffe aus diesen Substanzklassen umfassen bzw. aus einem oder mehreren dieser bestehen. Beispiele dafür wären:

Polyvinylacetat, Polyethylenglycol, Polyvinylalkohol, Cellulose und deren Ether und Ester, wie beispielsweise Cellulose-Pulver, mikrokristalline Cellulose, Ethylcellulose, Carboxymethylcellulose, Hydroxyethylcellulose, Hydroxypropylcellulose, Gummi arabicum, Carrageenan, Gelatine, Tragant, Amylose, Maltodextrine, Polysaccharide wie etwa Guargummi oder Alginate, Stärke, modifizierte Stärke, Pektine, Zuckeralkohole oder Kombinationen oder Copolymerisate davon. Bevorzugt ist Polyvinylacetat, beispielsweise in Form des kommerziell erhältlichen Kollidon^{®}, besonders bevorzugt Kollidon^{®} SR. Gemäß Herstellerangaben ist Kollidon^{®} SR ein Gemisch aus 80 % Polyvinylacetat und 19 % Povidon (Polyvinylpyrrolidon) sowie 0,8% Natriumlaurylsulfat und 0,6% Silica als Stabilisatoren.

Bevorzugt enthält beziehungsweise ist der Matrixbildner jedoch kein Polyacrylat oder sonstiges Polymer auf Basis eines Derivats der Acrylsäure oder Methacrylsäure.

Bevorzugt liegt bei polymeren Matrixbildnern das gewichtsmittlere Molekulargewicht im Bereich von 1*10³ bis 1*10⁷ g/mol, insbesondere bei mindestens 2.5*10³ g/mol. Es ist ferner bevorzugt, dass die Viskosität einer 2%-igen (w/w) wässrigen Lösung des Matrixbildners bei 25°C im Bereich von 30 bis 10.000 mPa·s, beispielsweise im Bereich von mindestens 2.000 oder 4.500 mPa·s liegt.

Der Matrixbildner ist bevorzugt in einer Menge von 5 bis 45 Gew.-% der oralen Darreichungsform enthalten, beispielsweise in einer Menge von 15 bis 40 Gew.-% oder 20 bis 35 Gew.-%.

Das Quellmittel kann beispielsweise einen oder mehrere der folgenden Stoffe umfassen oder aus einem oder mehreren von diesen bestehen:
Polyethylenoxid bzw. Polyethylenglykol, bevorzugt nicht-ionogene Polyethylenglykole; Cellulosederivate, beispielsweise Celluloseester oder -ether, z.B. Hydroxyalkylcellulose wie etwa Hydroxyethylcellulose, Hydroxypropylcellulose, Hydroxypropylmethylcellulose, Carboxymethylcellulose, Methylcellulose; Polyvinylalkohol; Polyvinylpyrrolidon; Carrageenan; Pektine; Alginate; kolloidale Magnesium-Aluminium-Silikate.

Besonders bevorzugt sind wasserlösliche Harze auf Basis nicht-ionogener Polyethylenglykole. Die Viskosität einer 2%-igen (w/w) wässrigen Lösung der bevorzugt nicht-ionogenen Polyethylenglykole bei 25°C liegt bevorzugt im Bereich von 1000 mPa·s, stärker bevorzugt 2000 bis 8000 mPa·s, stärker bevorzugt 2000 bis 4000 mPa·s Sie können beispielsweise ein gewichtsmittleres Molekulargewicht im Bereich von etwa 10⁵ bis 5*10⁶ g/mol aufweisen. Solche Polyethylenglykole sind beispielsweise unter der Handelsbezeichnung Polyox^{®} erhältlich, wobei hierein die Produkte mit der Handelsbezeichnung Polyox^{®} WSR N60K besonders bevorzugt sind.

Bevorzugt liegt bei den polymeren Quellmitteln allgemein das gewichtsmittlere Molekulargewicht im Bereich von 10³ bis 1.5*10⁶ g/mol, insbesondere bei mindestens 10⁴ g/mol. Es ist ferner bevorzugt, dass die Viskosität einer 2%-igen (w/w) wässrigen Lösung des Quellmittels bei 25°C im Bereich von 10.000 bis 80.000 mPa·s, beispielsweise im Bereich von mindestens 15.000 mPa·s oder mindestens 20.000 mPa·s liegt.

Das Quellmittel kann ferner wasserlöslich oder wasserunlöslich sein. Wasserunlöslich bezeichnet dabei eine Löslichkeit in deionisiertem Wasser von weniger als 33 mg/ml, während hierin alles als wasserlöslich bezeichnet wird, was darüber liegt, d.h. nicht was nicht wasserunlöslich ist.

Das Quellmittel ist bevorzugt in einer Menge von 10 bis 60 Gew.-% der oralen Darreichungsform enthalten, beispielsweise in einer Menge von 15 bis 60 Gew.-% oder 20 bis 60 Gew.-%.

Das Quellmittel selbst weist bevorzugt eine geringe beziehungsweise mäßige Quellgeschwindigkeit auf. Bevorzugt sind z.B. Quellmittel oder Quellmittelgemische, deren Volumen nach 10-minütigem Quellen in deionisiertem Wasser bei 37°C nicht mehr als 105%, bevorzugt nicht mehr als 110%, beispielweise nicht mehr als 120% oder 125% beträgt, verglichen mit dem trockenen Quellmittel vor dem Quellvorgang in Wasser.

Die orale Darreichungsform ist bevorzugt eine Tablette, insbesondere eine Matrixtablette, bevorzugt mit einer Größe von mindestens 9 mm, wie etwa mindestens 10 mm. Vorzugsweise beträgt eine größte Abmessung der oralen Darreichungsform im Querschnitt, beispielsweise der Matrixtablette, nach 30-minütigem Quellen in 0,1 M Salzsäure bei 37°C 9 mm oder mehr, beispielsweise mindestens 10, mindestens 11, mindestens 12 oder mindestens 13 mm. Die größte Abmessung im Querschnitt (lineare Entfernung) bezieht sich im Falle einer runden Tablette beispielsweise auf den Durchmesser (nicht den Umfang), bei einem Caplet (einer kapselförmigen Tablette) die Länge usw.. Vorzugsweise beträgt eine größte Abmessung der oralen Darreichungsform im Querschnitt nach 60-minütigem Quellen in 0,1 M Salzsäure bei 37°C mindestens 10, mindestens 11, mindestens 12 oder mindestens 13 mm.

Als Maß für das Quellvermögen wird der sogenannte Quellindex ermittelt. Dazu wird die Vergrößerung der oralen Darreichungsform, beispielsweise der Tablette gemessen, nachdem diese in einem 2000 mL-Becherglas in 1 Liter 37°C warme 0,06 N Salzsäure eingebracht wurde. Die orale Darreichungsform wird in regelmäßigen Abständen entnommen und ihre Abmessung mit einer Schiebelehre gemessen. Bei nicht rotationssymmetrischen oralen Darreichungsformen wird die größte (lineare) Dimension zugrunde gelegt und gemessen, beispielsweise bei Caplets die Länge. Bei runden (üblicherweise scheibenförmigen) Tabletten wird deren Durchmesser bestimmt.

Quellmittel und Matrixbildner, allein oder in Kombination, bewirken darüber hinaus vorzugsweise die modifizierte, besonders bevorzugt eine verlängerte Freisetzung des Pregabalins aus der oralen Darreichungsform. Es sind jedoch auch Ausführungsformen umfasst, in denen die orale Darreichungsform weitere Bestandteile enthält, die die Freisetzung des Pregabalins (ggf. zusätzlich) beeinflussen. Es können einerseits der Matrix weitere Bestandteile zugegeben werden, die das Freisetzungsverhalten beeinflussen beziehungsweise steuern. Es ist ferner denkbar, dass Pregabalin in einer Form eingesetzt wird, die nur langsam löslich ist oder dergleichen.

Bevorzugt sind orale Darreichungsformen mit einem Freisetzungsverhalten, gemessen mit USP Testapparatur II unter Verwendung von 900 ml 0,06 N HCl mit pH 1,4 unter Verwendung einer Drehgeschwindigkeit von 50 Umdrehungen pro Minute und bei einer Temperatur von 37°C, welches mindestens eine, bevorzugt zwei, mehr bevorzugt drei oder am stärksten bevorzugt alle der folgenden Bedingungen erfüllt:

| **Zeit [Minuten]** | **% freigesetztes Pregabalin** |
|---|---|
| 30 | 5 - 20 |
| 60 | 10 - 25 |
| 90 | 15 - 30 |
| 120 | 20 - 40 |
| 320 | 30 - 60 |
| 560 | 50 - 80 |
| 720 | 60 - 90 |

Neben den vorstehend genannten Bestandteilen kann die orale Darreichungsform beispielsweise einen oder mehrere zusätzliche Bestandteile enthalten, beispielsweise einen Gelbildner, beispielsweise zur Steuerung (ggf. Mitsteuerung) der Freisetzung. Als Gelbildner kommen etwa pharmazeutisch verträglichen Hydrogel- bzw. Hydrokolloidbildner in Frage, beispielsweise Bentonite, Tragant, Xanthan, Cellulosederivate wie z.B. Cellulosether oder -ester, z.B. Methylcellulose, Hydroxyalkylcellulose, insbesondere Hydroxyethylcellulose, Carrageenane, Polysaccharide, Guargummi, Ceraonia und besonders bevorzugt Polyacrylsäure, wie beispielsweise Carbomere (Carbopole). Carbopole sind (Homo- oder Co-)Polymere von Acrylsäure mit großer relativer Molmasse, die quervernetzt sind mit Polyalkenethern von Zuckern oder Polyalkoholen. Es können beispielsweise solche Carbomere eingesetzt werden, in denen die Polyacrylsäure (Homopolymer) mit Allylsucrose oder Allylpentaerythritol quervernetzt sind. Bevorzugt ist Carbopol^{®} 71 G.

Es kann auch ein Gemisch von Gelbildnern verwendet werden. Wird hierein Bezug genommen auf einen Gelbildner, so wird dieser als von Matrixbildner und Quellmittel verschieden, also als zusätzlicher Hilfsstoff, betrachtet. Das soll nicht bedeuten, dass Matrixbildner und/oder Quellmittel keine gelbildenden Eigenschaften haben könnte(n).

Die Viskosität einer 2%-igen (w/w) wässrigen Lösung des Gelbildners liegt bei 25°C im Bereich von 200 bis 45.000 m Pa·s, beispielsweise im Bereich von mindestens 10.000 oder mindestens 16.000 mPa·s.

Der Gelbildner ist, soweit enthalten, bevorzugt in einer Menge von 0 bis 25 Gew.-% der oralen Darreichungsform enthalten, beispielsweise in einer Menge von 5 bis 25 Gew.% oder 5 bis 20 Gew.-%.

Grundsätzlich umfasst im Rahmen dieser Anmeldung der Begriff "Pregabalin" sowohl die eingangs dargestellte "freie Aminosäure" (die zwitterionisch vorliegt) als auch pharmazeutisch verträgliche Salze, Solvate, Komplexe und Polymorphe davon. Pregabalin kann ferner auch als Racemat eingesetzt werden, bevorzugt jedoch als Enantiomer. Hierbei kann es sich um ein oder mehrere Salze handeln, die auch im Gemisch vorliegen können. Beispielhaft sind zu nennen die Säureadditionssalze anorganischer und/oder organischer Säuren, z.B. Hydrochloride, Carbonate, Hydrogencarbonate, Acetate, Lactate, Butyrate, Propionate, Sulfate, Methansulfonate, Citrate, Tartrate, Nitrate, Sulfonate, Oxalate und/oder Succinate; sowie Basenadditionssalze wie etwa solche der Alkali-oder Erdalkalimetallkationen oder Amine.

Bei dem verwendeten Pregabalin handelt es sich üblicherweise um kristallines Material, es kann jedoch auch in teilamorpher oder amorpher Form eingesetzt werden. Mengenangaben zu Pregabalin hierin beziehen sich auf die freie Aminosäure ohne etwaig vorhandenem Hydrat- beziehungsweise Solvatanteil.

Die erfindungsgemäße orale Darreichungsform enthält beispielsweise 10 bis 1000 mg Pregabalin, beispielsweise zwischen 50 und 600 mg Pregabalin. Pregabalin macht beispielsweise eine Menge von 2 Gew.-% bis 50 Gew.-% der oralen Darreichungsform aus.

Neben den oben genannten Bestandteilen kann die orale Darreichungsform weitere pharmazeutisch verträgliche Hilfsstoffe umfassen, beispielsweise Fließregulierungsmittel. Fließregulierungsmittel sind besonders bevorzugt, wenn die orale Darreichungsform in Form einer Tablette vorliegt. Sie haben die Aufgabe, in einem Tablettiergemisch sowohl die interpartikuläre Reibung (Kohäsion) zwischen den einzelnen Partikeln als auch das Haften dieser an den Wandflächen der Pressform (Adhäsion) zu vermindern. Ein Beispiel für einen Zusatz zur Verbesserung der Pulverfließfähigkeit ist disperses beziehungsweise kolloidales Siliciumdioxid (z.B. Aerosil^{®}). Bevorzugt wird Siliciumdioxid mit einer spezifischen Oberfläche von 50 bis 400 m²/g, bestimmt nach Gasadsorption gemäß Ph. Eur., 6. Auflage 2.9.26., verwendet.

In einer weiteren Ausführungsform kann die orale Darreichungsform, insbesondere bei Vorliegen in Tablettenform, beispielsweise zusätzlich Schmiermittel enthalten. Schmiermittel dienen im Allgemeinen der Verringerung der Gleitreibung. Insbesondere soll die Gleitreibung vermindert werden, die beim Tabletttieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat, (Pruv^{®}), Magnesiumstearat, Calciumstearat oder Gemische davon dar.

Ferner kann die orale Darreichungsform ein Netzmittel enthalten. Netzmittel haben die Aufgabe, die Benetzbarkeit von Wirk- und/oder Hilfsstoffen zu verbessern. Netzmittel sind beispielsweise anionische, kationische, amphotere oder nicht-ionische Tenside. Verwendet werden können beispielsweise folgende Tenside beziehungsweise Vertreter der folgenden Tensidklassen: Polyoxyethylen-Fettalkoholether, z.B. Macrogollaurylether, (z.B. Brij^{®}), ethoxylierte Sorbitanfettsäureester (auch bezeichnet als Polyoxyethylen-Sorbitanfettsäureester, z.B. Tween^{®}), Polyoxyethylen-Fettsäureglyceride, Polyoxyethylen-Fettsäureester, wie z.B. Macrogolstearat 400, Saccharose-Fettsäureester, nicht-ionische makromolekulare Tenside, wie z.B. Poloxamere, Natiumlaurylsulfat (auch bezeichnet als Natriumdodecylsulfat), Natriumcetylstearylsulfat, Phospholipide, ethoxyliertes Ricinusöl, Sojalecithin und andere, sowie Mischungen zweier oder mehr der vorstehend genannten Tenside.

Die orale Darreichungsform kann die oben genannten optionalen pharmazeutisch verträglichen Hilfsstoffe enthalten oder aber frei von diesen sein.

Unter einem weiteren Aspekt betrifft die Erfindung allgemein eine orale Darreichungsform zur modifizierten Freisetzung von Pregabalin, umfassend Pregabalin in einer quellfähigen Matrix, wobei ein Volumen der oralen Darreichungsform nach 10-minütigem Quellen in deionisiertem Wasser bei 37°C höchstens 30% größer ist als ein Volumen der Darreichungsform vor dem Quellen und wobei ein Volumen der oralen Darreichungsform nach 45-minütigem Quellen in 0,1 M Salzsäure bei 37°C mindestens 30% größer ist als ein Volumen der Darreichungsform vor dem Quellen.

Die erfindungsgemäße orale Darreichungsform ist in bevorzugten Ausführungsformen zur Verabreichung einmal täglich geeignet.

Dabei dient die erfindungsgemäße orale Darreichungsform bevorzugt zur Vorbeugung und/oder Behandlung, besonders bevorzugt der Behandlung, einer auf Pregabalin ansprechenden Krankheit oder auf Pregabalin ansprechenden Beschwerden. Die Krankheit ist, beziehungsweise die Beschwerden können beispielsweise Epilepsie, neuropathische Schmerzen, generalisierte Angststörung und/oder Fibromyalgie sein. Bei der Vorbeugung und/oder Behandlung von neuropathischen Schmerzen sind das z.B. neuropathische Schmerzen im Zusammenhang mit diabetischer Polyneuropathie, Post-Zoster-Neuralgie, Tumoren, Chemotherapie, Trigeminusneuralgie, Alkoholmissbrauch, Vitamin-B-Mangel, Phantomschmerz, Borrelien-Infektion, komplexes regionales Schmersyndrom, Engpasssyndrome, Rückenschmerzen und/oder AIDS. Daneben kommen auch die Vorbeugung und/oder Behandlung des Syndroms der ruhelosen Beine, der bipolaren Störung, von Migräne und Entzugserscheinungen in Betracht.

Erfindungsgemäße orale Darreichungsformen, insbesondere Matrixtabletten, weisen bevorzugt eine Masse von 150 bis 2000 mg, bevorzugt 200 bis 1000 mg, oder besonders bevorzugt 250 bis 800 mg auf.

Im Rahmen der Erfindung können die resultierenden Tabletten beschichtet oder unbeschichtet (befilmt oder unbefilmt) vorliegen. Als Filmbildner für die Beschichtung können beispielsweise Cellulosederivate wie etwa Methylcellulose (MC), Ethylcellulose (EC), Hydroxyethylcellulose (HEC), Methacrylsäure-Acrylat-Copolymere wie beispielsweise Methacrylsäure-Ethacrylat-Copolymer oder Methacrylsäure-Methylmethacrylat-Copolymer, Vinylpolymere wie beispielsweise Polyvinylpyrrolidon oder Polyvinylacetatphthalat oder natürliche Filmbildner, wie beispielsweise Schellack, verwendet werden. Die Dicke der Schicht, soweit vorhanden, beträgt üblicherweise 0,1 bis 100 µm, bevorzugt 1 bis 80 µm.

Bevorzugt werden die erfindungsgemäßen oralen Darreichungsformen ohne Befilmung beziehungsweise Beschichtung bereit gestellt.

Der Aufbau beziehungsweise das retentive Prinzip der erfindungsgemäßen oralen Darreichungsformen, insbesondere Tabletten, aus den vorstehend genannten Bestandteilen, ermöglicht das Erreichen einer vorteilhaften Härte. Insbesondere kann auch bei Anwendung eines vergleichsweise geringeren Pressdrucks bei der Kompression eine höhere Härte erzielt werden als im Stand der Technik. Beispielhafte Ausführungsformen weisen eine Härte von 50 bis 250 N, besonders bevorzugt mindestens 100 bis 230 N, insbesondere mindestens 150 N auf. Die Härte wird gemäß Ph.Eur. 6.0, Abschnitt 2.9.8 bestimmt.

Des Weiteren weisen die resultierenden Tabletten bevorzugt eine niedrige Friabilität auf, nämlich beispielsweise eine Friabilität von 0,1 bis 0,8 %, bevorzugt 0,2 bis 0,6 % und besonders bevorzugt 0,3 bis 0,5 %. Die Friabilität wird gemäß Ph.Eur. 6.0, Abschnitt 2.9.7 bestimmt.

Daneben ist Gegenstand der vorliegenden Erfindung ein Verfahren zur Herstellung der erfindungsgemäßen oralen Darreichungsform, umfassend eines der folgenden Verfahren:
(a) Mischen von Pregabalin, Matrixbildner, Quellmittel und gegebenenfalls einem oder mehreren pharmazeutisch verträglichen Hilfsstoffen und anschließende Kompression des resultierenden Gemischs zu einer Tablette,
(b) Granulation von Pregabalin, Matrixbildner, Quellmittel und gegebenenfalls einem oder mehreren pharmazeutisch verträglichen Hilfsstoffen zu einem Granulat, gegebenenfalls Zusatz eines oder mehrerer pharmazeutisch verträglicher Hilfsstoffe zu dem Granulat und anschließende Kompression zu einer Tablette.

Zu dem voranstehend genannten Verfahren gelten die Ausführungen in Bezug auf die orale Darreichungsform analog.

Bevorzugt ist Verfahren (a), die Direkttablettierung.

Unter "Granulieren" versteht man im Allgemeinen die Bildung gröberer oder körnigerer Haufwerke als Pulver durch Zusammenlagerung und/oder Aggregieren feinerer Pulverpartikel (Aufbaugranulation) und/oder die Bildung von feineren Granulaten durch Zerstückelung von gröberen Aggregaten (Abbaugranulation).

Üblicherweise kann Granulieren Feucht- oder Trockengranulieren bedeuten. Die Trockengranulierung erfolgt im Allgemeinen unter Anwendung von Druck oder Temperatur. Die Feuchtgranulation (Nassgranulation) erfolgt im Allgemeinen unter Verwendung von Oberflächenstabilisatoren und/oder Lösungs- bzw. Dispersionsmitteln. Das Granulieren erfolgt im Allgemeinen in üblichen Granuliervorrichtungen, wie beispielsweise Extruder-, Lochscheiben-, Lochwalzen-, oder Wirbelschichtgranulatoren. Ebenfalls können Zwangsmischer oder Sprühtrockner verwendet werden. Die Granulierung kann allgemein mit im Stand der Technik bekannten Verfahren erfolgen. Falls eine Feuchtgranulation durchgeführt wird, wird üblicherweise ein Schritt des "Trocknens" angewandt. Der Trockenschritt kann nach dem oder gleichzeitig mit dem Granulierschritt erfolgen. Unter "Trocknen" versteht man im Sinne dieser Erfindung die Abtrennung von an Feststoffen anhaftenden Flüssigkeiten. Trocknen erfolgt im Allgemeinen in üblichen Trocknungsvorrichtungen, beispielsweise Schrank-oder Hordentrockner, Vakuumtrockner, Wirbelschichttrockner, Sprühtrockner oder Gefriertrockner. Bevorzugt erfolgt der Trocken- und Granuliervorgang in einem Gerät.

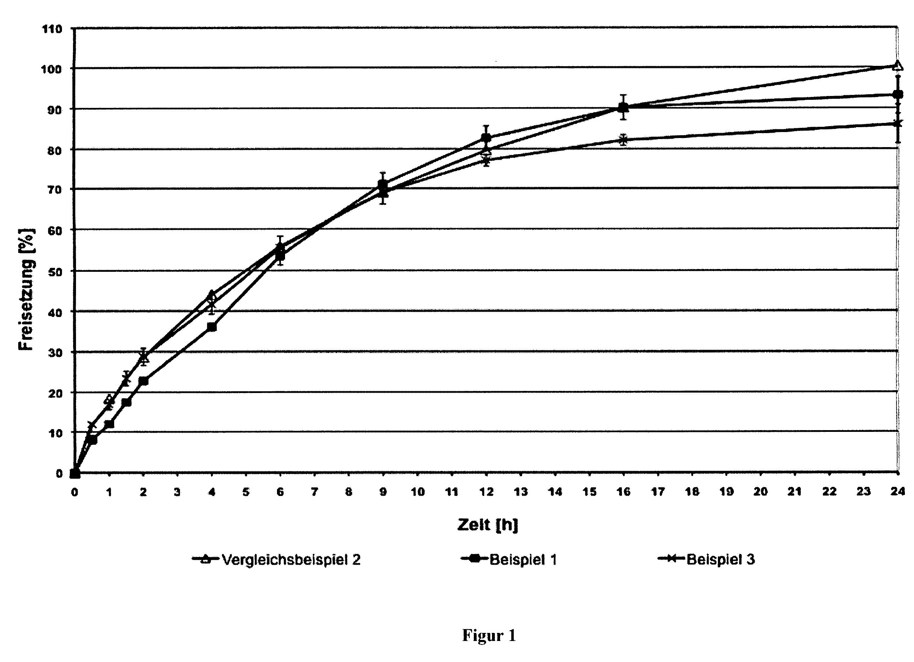

Die Erfindung wird durch nachstehende Beispiele unter Bezug auf die Figuren näher erläutert. Dabei zeigt
- **Figur 1**: die Freisetzungsprofile von Vergleichsbeispiel 2 sowie Beispielen 1 und 3.

### BEISPIELE

### Vergleichsbeispiel 1:

Zum besseren Vergleich mit dem Stand der Technik wurde Beispiel 30 der internationalen Patentanmeldung WO 2007/052125 nachgearbeitet:

| **Komponente** | **Funktion** | **Menge [mg]** | **Menge [%]** |
|---|---|---|---|
| Pregabalin | Wirkstoff | 100,7 | 26,8 |
| Kollidon^{®} SR | Matrixbildner | 85,3 | 22,7 |
| Plasdone XL^{®} | Quellmittel | 93,7 | 25,0 |
| Polyox^{®} WSR N60K NF | Quellmittel | 75,0 | 20,0 |
| Carbopol^{®} 71G | Gelbildner | 18,7 | 5,0 |
| Magnesiumstearat | Schmiermittel | 1,9 | 0,5 |
| | Σ | 375,3 | 100,0 |

Pregabalin, Kollidon^{®} SR, Plasdone XL^{®}, Polyox^{®} WSR N60K NF und Carbopol^{®} 71 G wurden in einem Freifallmischer (Turbula^{®} TB 10) 5 Minuten lang gemischt. Das erhaltene Gemisch wurde mit einem Sieb mit einer Maschenweite von 800 µm gesiebt. Das gesiebte Gemisch wurde dann erneut 10 Minuten lang in dem Freifallmischer gemischt. Magnesiumstearat wurde mit einem Sieb mit einer Maschenweite von 300 µm gesiebt und zu dem Gemisch hinzugegeben, dann wurde erneut 5 Minuten lang in dem Freifallmischer gemischt. Anschließend wurde das Gemisch mit Hilfe einer Exzenterpresse (Korsch^{®} EK0) mit 12 mm bikonvexen Pressstempeln und einem Pressdruck von 24 kN zu einer Tablette mit den folgenden Eigenschaften verpresst:

| **Pressdruck [kN]** | **Härte [N]** | **Durchmesser [mm]** | **Dicke [mm]** |
|---|---|---|---|
| 24 | 150 | 12,1 | 4,65 |

Die Messung des Quellindex ergab das in der nachfolgenden Tabelle dargestellte Quellverhalten (1L 37 °C warme 0,06 N HCl in 2000 mL Becherglas, Messung des Durchmessers mit Hilfe einer Schiebelehre):

| **Zeit [Stunden]** | **Durchmesser [mm]** | **% Zunahme** |
|---|---|---|
| 0 | 12,1 | |
| 1 | 17,0 | 40,5 |
| 2 | 17,0 | 40,5 |
| 4 | 16,5 | 36,4 |
| 8 | 16,5 | 36,4 |
| 24 | 17,0 | 40,5 |

Die Quellung auf die maximale Größe erfolgte mithin innerhalb der ersten Stunde. Der Durchmesser hat dabei um 40,5% zugenommen.

### Vergleichsbeispiel 2:

Vergleichsbeispiel 1 wurde mit den gleichen Mengen und Verfahrensschritten wiederholt, mit Ausnahme der Kompressionsbedingungen. Das Gemisch wurde in Vergleichsbeispiel 2 mit einem Pressdruck von 20 kN zu einer Tablette mit den nachfolgend aufgelisteten Eigenschaften verpresst.

| **Pressdruck [kN]** | **Härte [N]** | **Durchmesser [mm]** | **Dicke [mm]** |
|---|---|---|---|
| 20 | 110 | 11,8 | 4,4 |

Die Messung des Quellindex ergab das in der nachfolgenden Tabelle dargestellte Quellverhalten (Bedingungen wie zuvor):

| **Zeit [Minuten]** | **Durchmesser [mm]** | **% Zunahme** |
|---|---|---|
| 0 | 11,8 | |
| 5 | 19,6 | 66,0 |
| 10 | 18,0 | 52,4 |
| 30 | 15,2 | 28,7 |

Diese Messung belegt, dass bereits nach 5 Minuten eine maximale Quellung erreicht ist, die Tabletten also sehr schnell quellen.

In Figur 1 ist das Freisetzungsprofil von Vergleichsbeispiel 2 dargestellt.

### Beispiel 1: Matrixtablette mit Auftriebsmittel

| **Komponente** | **Funktion** | **Menge [mg]** | **Menge [%]** |
|---|---|---|---|
| Pregabalin | Wirkstoff | 100,7 | 22,34 |
| Kollidon^{®} SR | Matrixbildner | 100,0 | 22,19 |
| Natriumhydrogencarbonat | Auftriebsmittel | 43,0 | 9,54 |
| Polyox^{®} WSR N60K NF | Quellmittel | 116,0 | 25,74 |
| Carbopol^{®} 71G | Gelbildner | 89,0 | 19,75 |
| Magnesiumstearat | Schmiermittel | 2,0 | 0,44 |
| | Σ | 450,7 | 100,0 |

Pregabalin, Kollidon^{®} SR, Natriumhydrogencarbonat, Polyox^{®} WSR N60K NF und Carbopol^{®} 71 G wurden in einem Freifallmischer (Turbula^{®} TB 10) 5 Minuten lang gemischt. Das erhaltene Gemisch wurde mit einem Sieb mit einer Maschenweite von 800 µm gesiebt. Das gesiebte Gemisch wurde dann erneut 10 Minuten lang in dem Freifallmischer gemischt. Magnesiumstearat wurde mit einem Sieb mit einer Maschenweite von 300 µm gesiebt und zu dem Gemisch hinzugegeben, dann wurde erneut 5 Minuten lang in dem Freifallmischer gemischt. Anschließend wurde das Gemisch mit Hilfe einer Exzenterpresse (Korsch^{®} EK0) mit 12 mm bikonvexen Pressstempeln und einem Pressdruck von 20 kN zu einer Tablette mit den nachfolgend aufgelisteten Eigenschaften verpresst.

| **Pressdruck [kN]** | **Härte [N]** | **Durchmesser [mm]** | **Dicke [mm]** |
|---|---|---|---|
| 20 | 192 | 12,0 | 4,8 |

Bei gleichem Pressdruck haben die erfindungsgemäßen Tabletten eine deutlich bessere Härte als Vergleichsbeispiel 2 (110 N).

Die Messung des Quellindex (Bedingungen wie zuvor) ergab das in der nachfolgenden Tabelle dargestellte Quellverhalten:

| **Zeit [Stunden]** | **Durchmesser [mm]** | **% Zunahme** |
|---|---|---|
| 0 | 12,0 | |
| 1 | 13,5 | 11,7 |
| 2 | 14,2 | 18,1 |
| 4 | 15,0 | 24,4 |
| 8 | 15,2 | 26,0 |
| 24 | 19,0 | 57,8 |

Die Quellung erfolgte erheblich langsamer als in den beiden Vergleichsbeispielen und zog sich über 24 Stunden hin.

Ferner stieg die Tablette innerhalb von 30 Sekunden auf (1L 37 °C warme 0,06 N HCl in 2000 mL Becherglas, Messung des Durchmessers mit Hilfe einer Schiebelehre).

Trotz unterschiedlichen Quellverhaltens kann mit Beispiel 1 etwa das gleiche Freisetzungsverhalten erreicht werden wie mit Vergleichsbeispiel 2. Dies ist aus einem Vergleich der Freisetzungsprofile in Figur 1 ersichtlich.

### Beispiel 2: Matrixtablette mit Auftriebsmittel

Beispiel 1 wurde wiederholt, wobei eine Tablette mit den nachfolgend aufgelisteten Eigenschaften erhalten wurde:

| **Pressdruck [kN]** | **Härte [N]** | **Durchmesser [mm]** | **Dicke [mm]** |
|---|---|---|---|
| 20 | 200 | 12,1 | 4,5 |

Die Messung des Quellindex (Bedingungen wie zuvor) ergab das in der nachfolgenden Tabelle dargestellte Quellverhalten:

| **Zeit [Minuten]** | **Durchmesser [mm]** | **% Zunahme** |
|---|---|---|
| 0 | 12,1 | |
| 5 | 13,2 | 9,1 |
| 10 | 13,3 | 10,8 |
| 30 | 13,5 | 11,6 |

Die Quellung erfolgt mithin erheblich langsamer als in den Vergleichsbeispielen. Während im Vergleichsbeispiel (2) das finale Quellvolumen praktisch nach 5 Minuten erreicht ist, nimmt das Quellvolumen und damit der Quellindex in Beispiel 2 nur langsam zu.

### Beispiel 3: Matrixtablette mit Sedimentationsmittel

| **Komponente** | **Funktion** | **Menge [mg]** | **Menge [%]** |
|---|---|---|---|
| Pregabalin | Wirkstoff | 100,7 | 22,51 |
| Kollidon^{®} SR | Matrixbildner | 99,9 | 22,33 |
| Natriumchlorid | Sedimentationsmittel | 40,0 | 8,94 |
| Polyox^{®} WSR N60K NF | Quellmittel | 115,9 | 25,91 |
| Carbopol^{®} 71G | Gelbildner | 88,9 | 19,87 |
| Magnesiumstearat | Schmiermittel | 1,9 | 0,42 |
| | Σ | 447,3 | 100,0 |

Pregabalin, Kollidon^{®} SR, Natriumchlorid, Polyox^{®} WSR N60K NF und Carbopol^{®} 71 G wurden in einem Freifallmischer (Turbula^{®} TB 10) 5 Minuten lang gemischt. Das erhaltene Gemisch wurde mit einem Sieb mit einer Maschenweite von 800 µm gesiebt. Das gesiebte Gemisch wurde dann erneut 10 Minuten lang in dem Freifallmischer gemischt. Magnesiumstearat wurde mit einem Sieb mit einer Maschenweite von 300 µm gesiebt und zu dem Gemisch hinzugegeben, dann wurde erneut 5 Minuten lang in dem Freifallmischer gemischt. Anschließend wurde das Gemisch mit Hilfe einer Exzenterpresse (Korsch^{®} EK0) mit 12 mm bikonvexen Pressstempeln und einem Pressdruck von 21 kN zu einer Tablette mit den folgenden Eigenschaften verpresst:

| **Pressdruck [kN]** | **Härte [N]** | **Durchmesser [mm]** | **Dicke [mm]** |
|---|---|---|---|
| 21 | 180 | 12 | 4,7 |

Auch Beispiel 3 hat eine deutlich bessere Härte als die Vergleichsbeispiele.

Die Messung des Quellindex ergab das in der nachfolgenden Tabelle dargestellte Quellverhalten:

| **Zeit [Stunden]** | **Durchmesser [mm]** | **% Zunahme** |
|---|---|---|
| 0 | 11,9 | |
| 1 | 13,7 | 15,1 |
| 2 | 15,0 | 26,4 |
| 3 | 15,2 | 27,9 |
| 4 | 15,2 | 28,5 |
| 8 | 15,9 | 34,3 |
| 24 | 17,4 | 46,5 |

Die Quellung erfolgte erheblich langsamer als im Vergleichsbeispiel und zog sich über 24 Stunden hin.

Nach Einbringen in 1L 37 °C warme 0,06 N HCl in einem 2000 mL Becherglas sank die Tablette sogleich auf den Becherboden ab, die Sedimentation erfolgte also unmittelbar.

Das Freisetzungsprofil von Beispiel 3 ist in Figur 1 dargestellt. Auch hier kann trotz unterschiedlichen Quellverhaltens etwa das gleiche Freisetzungsverhalten erreicht werden wie mit Vergleichsbeispiel 2.

### Beispiel 4: Matrixtablette mit Sedimentationsmittel

Beispiel 3 wurde wiederholt und lieferte eine Tablette mit folgenden Eigenschaften:

| **Pressdruck [kN]** | **Härte [N]** | **Durchmesser [mm]** | **Dicke [mm]** |
|---|---|---|---|
| 20 | 180 | 12 | 4,6 |

Auch diese Tablette sank nach Einbringen in 1L 37 °C warme 0,06 N HCl in einem 2000 mL Becherglas sogleich auf den Becherboden ab

Die Messung des Quellindex ergab das in der nachfolgenden Tabelle dargestellte Quellverhalten in der ersten halben Stunde:

| **Zeit [Minuten]** | **Durchmesser [mm]** | **% Zunahme** |
|---|---|---|
| 0 | 12,1 | |
| 5 | 12,9 | 6,6 |
| 10 | 13,2 | 9,1 |
| 30 | 13,3 | 10,8 |

## Patentansprüche

1. Orale Darreichungsform zur modifizierten Freisetzung von Pregabalin, umfassend Pregabalin in einer Matrix, die ein Quellmittel, einen Matrixbildner und ein Auftriebsmittel oder alternativ ein Sedimentationsmittel umfasst.

2. Orale Darreichungsform nach Anspruch 1, wobei ein Volumen der oralen Darreichungsform nach 10-minütigem Quellen in deionisiertem Wasser bei 37°C maximal 30% größer ist als ein Volumen der Darreichungsform vor dem Quellen.

3. Orale Darreichungsform nach Anspruch 1 oder 2, wobei das Auftriebsmittel geeignet ist, bei Kontakt mit 0,1 M Salzsäure ein Gas, bevorzugt Kohlendioxid und/oder Stickstoff freizusetzen.

4. Orale Darreichungsform nach Anspruch 3, wobei das Auftriebsmittel ausgewählt ist aus der Gruppe bestehend aus Carbonaten, Hydrogencarbonaten, Citronensäure, Ascorbinsäure, Weinsäure und Kombinationen davon.

5. Orale Darreichungsform nach einem der Ansprüche 1 bis 2, wobei das Sedimentationsmittel ausgewählt ist aus der Gruppe anorganischer Salze, bevorzugt ein Alkali- oder Erdalkalichlorid.

6. Orale Darreichungsform nach einem der vorangehenden Ansprüche, wobei das Quellmittel ein Polyethylenglykol, bevorzugt nicht-ionogenes Polyethylenglykol, Cellulosederivat wie Celluloseester oder -ether, Polyvinylalkohol, Polyvinylpyrrolidon, Carrageenan, Pektin, Alginat, kolloidales Magnesium-Aluminium-Silikat oder eine Kombination davon umfasst.

7. Orale Darreichungsform nach einem der vorangehenden Ansprüche, wobei der Matrixbildner Polyvinylacetat, ein Gemisch von Polyvinylacetat und Polyvinylpyrrolidon, Polyethylenglycol, Polyvinylalkohol, Cellulose, Celluloseether, Celluloseester, Gelatine, Gummi Arabicum, Carrageenan, Gelatine, Tragant, Amylose, Maltodextrine, Polysaccharide, Stärke, modifizierte Stärke, Pektin, Zuckeralkohol oder Kombinationen umfasst.

8. Orale Darreichungsform nach einem der vorangehenden Ansprüche, wobei eine Abmessung der oralen Darreichungsform nach 30-minütigem Quellen in 0,1 M Salzsäure bei 37°C 9 mm oder mehr beträgt.

9. Orale Darreichungsform nach einem der vorangehenden Ansprüche mit einem Freisetzungsverhalten, gemessen mit USP Testapparatur II unter Verwendung von 900 ml 0,06 N HCl mit pH 1,4 unter Verwendung einer Drehgeschwindigkeit von 50 Umdrehungen pro Minute und bei einer Temperatur von 37°C, welches mindestens eine der folgenden Bedingungen erfüllt:
| Zeit [Minuten] | % freigesetztes Pregabalin |
|---|---|
| 30 | 5 - 20 |
| 60 | 10 - 25 |
| 90 | 15 - 30 |
| 120 | 20 - 40 |
| 320 | 30 - 60 |
| 560 | 50 - 80 |
| 720 | 60 - 90 |

10. Orale Darreichungsform nach einem der vorangehenden Ansprüche zur Verabreichung einmal täglich.

11. Orale Darreichungsform nach einem der vorangehenden Ansprüche, enthaltend 50 bis 600 mg Pregabalin.

12. Orale Darreichungsform nach einem der vorangehenden Ansprüche zur Vorbeugung oder Behandlung einer auf Pregabalin ansprechenden Krankheit oder auf Pregabalin ansprechende Beschwerden.

13. Orale Darreichungsform nach Anspruch 12, zur Vorbeugung oder Behandlung von Epilepsie, neuropathischen Schmerzen, generalisierter Angststörung und/oder Fibromyalgie, insbesondere neuropathischen Schmerzen im Zusammenhang mit diabetischer Polyneuropathie, Post-Zoster-Neuralgie, Tumoren, Chemotherapie, Trigeminusneuralgie, Alkoholmissbrauch, Vitamin-B-Mangel, Phantomschmerz, Borrelien-Infektion, komplexem regionalem Schmerzsyndrom, Engpasssyndromen, Rückenschmerzen und/oder AIDS.

14. Verfahren zur Herstellung einer oralen Darreichungsform gemäß einem der vorangehenden Ansprüche, umfassend:
(a) Mischen von Pregabalin, Matrixbildner, Quellmittel und gegebenenfalls einem oder mehreren pharmazeutisch verträglichen Hilfsstoffen und anschließende Kompression des resultierenden Gemischs zu einer Tablette, oder
(b)Granulation von Pregabalin, Matrixbildner, Quellmittel und gegebenenfalls einem oder mehreren pharmazeutisch verträglichen Hilfsstoffen zu einem Granulat, gegebenenfalls Zusatz eines oder mehrerer pharmazeutisch verträglicher Hilfsstoffe zu dem Granulat und anschließende Kompression zu einer Tablette.
